# EUROPEAN PATENT APPLICATION

(11) **EP 2 698 156 A1**
(43) Date of publication of application: **19.02.2014**
(21) Application number: 12005900.1
(22) Date of filing: 16.08.2012
(51) Int. Cl.: A61K 31/192, A61P 35/00

(54) **Phenylbutyric acid for chemoprevention**

(71) Applicant: Lunamed AG, 7000 Chur (CH)
(72) Inventor: Truog, Peter, 7000 Chur (CH); Buschmann, Helmut H., 52076 Aachen (Walheim) (DE)
(74) Representative: Peters, Hajo

(57) **Abstract**

The invention provides a pharmaceutical composition comprising phenylbutyric acid, a derivative, a prodrug, solvate or a physiologically acceptable salt thereof for use in the prevention of cancer. Furthermore pharmaceutical compositions are disclosed which comprise a further chemopreventive agent.

## Description

### Field of the invention

The present invention refers to a pharmaceutical composition comprising phenylbutyric acid, a derivative, codrug, co-crystal or a physiologically acceptable salt thereof for use in the prevention of cancer. In a further aspect the invention relates to a pharmaceutical composition wherein the phenylbutyric acid, its derivative, or salt is combined with at least one additional chemopreventive agent in order to yield a codrug, co-crystal or combination.

### Background of the invention

Cancer can be defined as an abnormal growth of tissue characterized by a loss of cellular differentiation. This term encompasses a large group of diseases in which there is an invasive spread of undifferentiated cells from a primary site to other parts of the body where further undifferentiated cellular replication occurs, which eventually interferes the normal functioning of tissues and organs.

Cancer is primarily an environmental disease with 90-95% of cases attributed to environmental factors and 5-10% due to genetics. Environmental, as used by cancer researchers, means any cause that is not inherited genetically, not merely pollution. Common environmental factors that contribute to cancer death include tobacco (25-30%), diet and obesity (30-35%), infections (15-20%), radiation (both ionizing and non-ionizing, up to 10%), stress, lack of physical activity and environmental pollutants.

With more than 3 million new cases and 1.7 million deaths each year, cancer represents the second most important cause of death and morbidity in Europe. On a global scale, cancer accounted for 7.4 million deaths (around 13% of the total) in 2004.

Although more than 40% of cancer deaths can be prevented, cancer is a leading cause of death, causing 20% of the total in the European Region. Noticeably, Europe comprising only one eighth of the total world population but has around one quarter of the global total of cancer cases: some 3.2 million new patients per year.

The most common forms of cancer were prostate, colorectal, breast and lung cancer. The risk of getting cancer before the age of 75 years is 26.5%, or around one in four. However, because the population of Europe is ageing, the rate of new cases of cancer is also expected to increase.

Cancer can be defined by four characteristics which differentiate neoplastic cells from normal ones: (1) clonality - cancer starts from genetic changes in a single cell which multiplies to form a clone of neoplastic cells; (2) autonomy - biochemical and physical factors that normally regulate cell growth, do not do so in the case of neoplastic cells; (3) anaplasia - neoplastic cells lack normal differentiation which occurs in nonmalignant cells of that tissue type; (4) metastasis - neoplastic cells grow in an unregulated fashion an spread to other parts of the body.

Each cancer is characterized by the site, nature and clinical cause of undifferentiated cellular proliferation. The underlying mechanism for the initiation of cancer is not completely understood; however, about 80% of cancers may be triggered by external stimuli such as exposure to certain chemicals, tobacco smoke, ultra violet rays, ionizing radiation, and viruses. Development of cancer in immunosuppressed individuals indicates that the immune system is an important factor controlling the replication and spread of cancerous cells throughout the body. The high incidence of cancer in certain families, though, suggests a genetic disposition towards development of cancer. The molecular mechanisms involved in such genetic dispositions fall into a number of classes including those that involve oncogenes and suppressor genes.

Proto-oncogenes are genes that code for growth promoting factors necessary for normal cellular replication. Due to mutation, such proto-oncogenes are inappropriately expressed and are then termed oncogenes. Oncogenes can be involved in malignant transformation of the cell by stimulating uncontrolled multiplication.

Suppressor genes normally act by controlling cellular proliferation through a number of mechanisms including binding transcription factors important to this process. Mutations or deletions in such genes contribute to malignant transformation of a cell. Malignant transformation develops and cancer results because cells of a single lineage accumulate defects in certain genes such as proto-oncogenes and suppressor genes responsible for regulating cellular proliferation. A number of such specific mutations and/or deletions must occur in a given cell for initiation of uncontrolled replication. It is believed that genetic predisposition to a certain type of cancer results from inheritance of genes that already have a number of mutations in such key regulatory genes and subsequent exposure to environmental carcinogens causes enough additional key mutations or deletions in these genes in a given cell to result in malignant transformation. Changes in other types of genes could further the ability of tumours to grow, invade local tissue, and establish metastases at distant body sites.

Current treatments of cancer and related diseases have limited effectiveness and numerous serious unintended side effects. Cancer therapy is currently divided into many categories: surgery, radiation therapy, bone marrow therapy, stem cell transplantation, chemotherapy, gene therapy, hormonal therapy, immunotherapy and antiangiogenic therapy. These treatments have progressed only incrementally during more than thirty years of intensive research to discover the origins of cancer and devise improved therapies for cancer and related diseases.

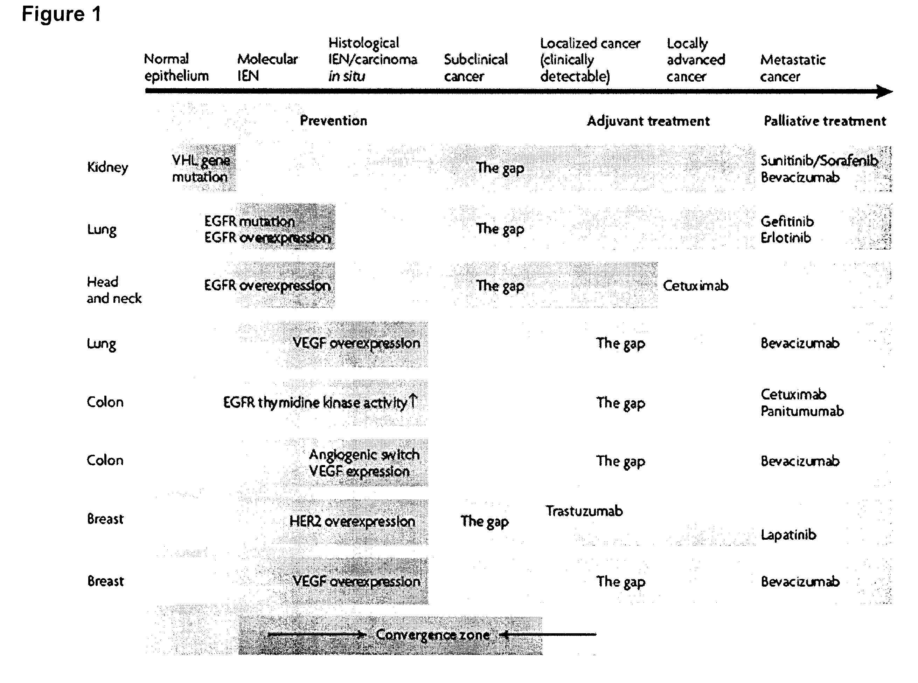

Molecular alterations occur early on during the process of carcinogenesis. However, agents targeted to these molecules are often incorporated into cancer treatment at a relatively late stage. The delay in pharmacologically addressing these molecular alterations is a critical gap and represents a window of opportunity for the development of molecularly targeted strategies for the prevention of cancer (see Figure 1).

Chemoprevention, i.e., the prevention of cancer by administration of chemical agents that reduce the risk of carcinogenesis is one of the most direct ways to reduce cancer-related morbidity and mortality. However, chemoprevention requires the identification of carcinogens and chemopreventives, even though interactions between the factors that modulate cancer risk are complex. Whereas extensive efforts have been made to identify carcinogens and mutagens, the identification of chemopreventive agents has received less attention. Cancer chemopreventive agents include nonsteroidal anti-inflammatory drugs (NSAIDs), such as indomethacin, aspirin, piroxicam, and sulindac, all of which inhibit cyclooxygenase.

There is a need in the art, however, for the identification of additional specific compounds that have a cancer chemopreventive effect on mammals. Such cancer chemopreventive compounds then can be used in drug compositions to reduce the risk of cancer or to delay the time until cancer occurs.

Accordingly, it was the goal of this invention to identify an alternative chemopreventive compound.

It has now been found that a pharmaceutical composition comprising phenylbutyric acid can be used for chemoprevention.

Accordingly, the invention is drawn to a pharmaceutical composition comprising phenylbutyric acid, a prodrug, a derivative, a codrug, a co-crystal, a pharmaceutically acceptable salt or solvate thereof; for the use in the prevention of cancer.

Phenylbutyric acid, especially 4-phenylbutyric acid as depicted in formula (I) is a histone deacetylase inhibitor that is known as a pharmaceutically active component for many years.

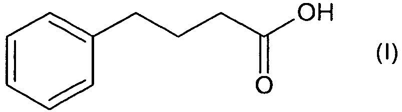

In form of its sodium salt it is marketed as a drug in the USA and European Union. Sodium phenylbutyrate is an orphan drug, marketed by Ucyclyd Pharma (Hunt Valley, USA) under the trade name Buphenyl and by Swedish Orphan International (Sweden) as Ammonaps for the treatment of urea cycle disorders.

As emphasized in a recent review by Davies and Wu (Discov. Med. 2012, 13(72): 385-390) the number of approved chemoprevention drugs is still quite small despite numerous chemoprevention trials in the past 10 years. According to Davies and Wu this is likely due to a number of factors, but two consistent problems in the field of chemoprevention have been the lack of efficacy with or without unacceptable toxicity, and unexpected toxicity or fear of it associated with most of the approaches.

4-phenylbutyric acid overcomes both problems. As a histone deacetylase inhibitor it possesses the required efficacy and as a compound with proven and established safety it lacks toxicity problems.

Histone deacetylases (HDACs) have been extensively studied for their roles in transcriptional regulation and chromatin remodelling. HDACs are divided into four classes, according to sequence homology and domain organization (Dokmanovic et al. (2007) Mol. Cancer Res. 5: 981-989). Class I includes, for example, HDACs 1, 2, 3, and 8; class IIa includes, for example, HDACs 4, 5 7 and 9; class IIb includes HDACs 6 and 10, class III includes HDACs SIRT 1, 2, 3, 4, 5, 6 and 7, and class IV includes HDAC11.

HDACs have also drawn research interest because inhibitors of these enzymes display anti-tumour activities (reviewed in Drummond et al. (2005) Ann. Rev. Pharmacol. Toxicol. 45: 495-528). Certain HDAC inhibitors (HDACi) are at various stages of clinical trials for cancer patients, and at least one (SAHA/vorinostat) has been approved for clinical use (Gallinari et al. (2007) Cell Res 17: 195-211).

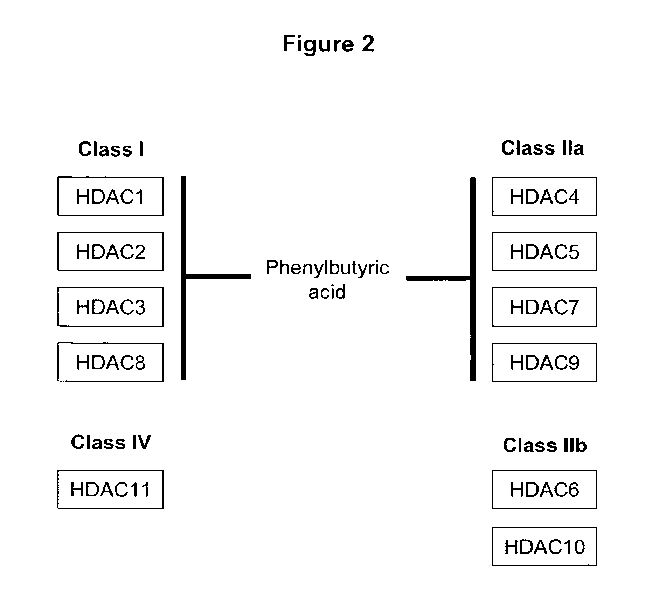

As depicted in Figure 2 the compound 4-phenylbutyric acid has been shown to inhibit a broad range of altogether 8 out of 18 known HDACs, namely HDAC1, 2, 3 and 8 from class I and HDAC 4, 5, 7 and 9 from class IIa. Noticeably, the HDACs from class III, the so-called sirtuins have not been found to be inhibited by phenylbutyric acids. Since these HDACs are discussed for a role in metabolism and longevity (resveratrol is a sirtuin activator) the lack of inhibition represents a beneficial selectivity for the phenylbutyric acids.

Histone deacetylase inhibitors such as phenylbutyric acids lower the apoptotic threshold of neoplastic cells. During transformation, neoplastic cells reach a state of apoptotic homeostasis whereby the activity of activated pro-apoptotic proteins is counterbalanced by elevated expression/activation of anti-apoptotic proteins to ensure tumour cell survival. It was found that neoplastic cells treated with the histone deacetylase inhibitor phenylbutyric acid generally upregulate pro-apoptotic genes involved in the death receptor pathway (for example, TRAIL and DR5) and/or the intrinsic apoptotic pathway (that is, Bax, Bak and APAF1), whereas prosurvival genes (that is, BCL2 and XIAP) are generally down regulated, thereby lowering the apoptotic threshold within the treated cell.

In addition to regulating the acetylation state of histones, histone deacetylase (HDAC) can bind to, deacetylate and regulate the activity of a number of other proteins, including transcription factors (e.g. p53, E2F transcription factor 1 (E2F1) and nuclear factor-KB (NF-κB)) and proteins with diverse biological functions (e.g. α-tubulin, Ku70 and heat-shock protein 90 (Hsp90)). Hyperacetylation of transcription factors with HDAC inhibitors (HDACi) can augment their gene-regulatory activities and contribute to the changes in gene expression observed following direct HDACi mediated histone hyperacetylation. Hyperacetylation of proteins such as Ku70 and Hsp90 or disruption of protein phosphatase 1 (PP1)-HDAC interactions by HDACi might have no direct or indirect effect on gene expression but could be important for certain biological effects of HDACi, in particular the induction of apoptosis and cell cycle arrest.

In sum the HDACi phenylbutyric acid can prevent cancerogenesis through multiple biological effects.

The scientific basis for the use of a histone deacetylase inhibitor is the progression of cells from a normal homogenous state to more and more heterogeneity as carcinogenesis evolves. Therefore, more cells can be affected by treatment in early stages of carcinogenesis when they are more homogenous than in later heterogeneous stages.

Since the histone deacetylases as targets for phenylbutyric acid represent a ubiquitously expressed protein family the pharmaceutical composition of the invention should be capable to prevent cancer from each cancer type.

Due its proven safety it can be given in enhanced doses required for patients with e.g. an enhanced risk of cancerogenesis.

It has to be emphasised that the lack of toxicity allows that the phenylbutyric acid can be given over a long period of time which is crucial for a chemopreventive agent.

Noticeably, phenylbutyric acid does not show relevant interactions with other drugs, so that the concomitant use of other medicaments does not preclude its use.

In addition there are no critical food interactions reported for phenylbutyric acid. This eases the application and therewith enhances the patient compliance.

Finally, non-responders for phenylbutyric acid have not reported e.g. due to differences in metabolism. This allows a broad application without selecting responsive patient subgroups.

As a relatively inexpensive compound, the phenylbutyric acid, especially when given chronically, represents an affordable prevention therapy and therefore is superior also from a pharmaco-economic point of view.

Noticeably, the profile of phenylbutyric acid is in line with the priorisation of chemopreventive agents as established by the NCI program which bases the priorities for candidate compounds on four factors: efficacy, toxicity, commercial availability; and possible mechanism of action.

### Detailed description of the invention

In the first aspect the invention provides a pharmaceutical composition comprising phenylbutyric acid, a derivative, a prodrug, codrug, co-crystal, solvate or a physiologically acceptable salt thereof for use in the prevention of cancer.

In a further preferred embodiment of the invention the phenylbutyric acid comprised within the pharmaceutical composition of the invention is 4-phenylbutyric acid.

In one embodiment of the invention the pharmaceutical composition comprises a prodrug of 4-phenylbutyric acid, which is preferably an ester, an amide or an anhydride.

In a preferred embodiment of the invention the 4-phenylbutyric acid prodrug is a carbonic acid anhydride, being preferably pivaloyloxymethyl-4-phenylbutyrate.

In another embodiment of the invention the prodrug is an ester of 4-phenylbutyric acid, which preferably is an ester with an alcohol selected from the group consisting of methanol, ethanol, trichloroethanol, propanol, n-butanol, isobutanol, 1-pentanol, 2-pentanol, 3-pentanol, 2-methyl-2-butanol, 2-methyl-1-butanol, 3-methy-1-butanol, 3-methyl-2-butanol, 2,2-dimethyl-1-propanol, 1-hexanol, 2-hexanol, 3-hexanol, 2-methyl-1-pentanol, 3-methyl-1-pentanol, 4-methyl-1-pentanol, 2-methyl-2-pentanol, 3-methyl-2-pentanol, 4-methyl-2-pentanol, 2-methyl-3-pentanol, 3-methyl-3-pentanol, 2,2-dimethyl-1-butanol, 2,3-dimethyl-1-butanol, 2,3-dimethyl-2-butanol, 3,3-dimethyl-2-butanol, 2-ethyl-1-butanol and 2-propyl-pentanol.

In a further embodiment of the invention the prodrug is a diethylcarbonate prodrug of 4-phenylbutyric acid.

In another embodiment of the invention the prodrug is an 4-phenylbutyric amide which is formed with an amine preferably selected from the group consisting of a naturally occurring amino acid such as alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine, being more preferably phenylalanine.

In a preferred embodiment the 4-phenylbutyrate prodrug is butyroyloxymethyl-4-phenylbutyrate.

In a preferred embodiment the pharmaceutical composition according to the invention comprises a pharmaceutically acceptable salt of 4-phenylbutyric acid whereby the pharmaceutically acceptable salt is preferably selected from the group consisting of sodium, potassium, ammonium, lithium calcium, magnesium, zinc, iron, monoethanolamine, diethanolamine, triethanolamine, lysine, arginine, and tromethamine. For a review of pharmaceutically acceptable salts see P. H. Stahl and C. G. Wermuth, editors, Handbook of Pharmaceutical Salts: Properties, Selection and Use, Weinheim/Zürich:Wiley-VCH/VHCA, 2002, incorporated herein by reference.

In a further preferred embodiment said salt is a physiologically acceptable salt of 4-phenyl-butyric acid of the earth or earth alkaline type, most preferably is a lithium, sodium, magnesium or calcium, and specifically the sodium salt of 4-phenyl-butyric acid.

In another aspect of the invention the pharmaceutical composition according to the invention is used for cancer prevention, wherein said prevention is a primary, secondary or tertiary prevention of cancer. In primary chemoprevention the phenyl butyric acid of the invention is capable to prevent the transition of normal cells to precancerous lesions. The precancerous lesions representing a preliminary stage of cancer can have several causes; UV radiation, genetics, exposure to such cancer-causing substances (carcinogens) as arsenic, tar or x-ray radiation. Furthermore, the phenylbutyric acid of the invention can be used for secondary chemoprevention which focuses on preventing the progression of these precancerous lesions to cancer and tertiary chemoprevention whereby the recurrence or spread of a primary cancer is prevented. Finally, the prevention using the pharmaceutical composition of the invention also encompasses the treatment of Carcinoma *in situ* (CIS).

In one embodiment of the invention the pharmaceutical composition according to the invention can be used prevent malignant as well as benign tumours.

In a further embodiment the cancer or malignant tumour, either primary or secondary, that can be prevented by the pharmaceutical compositions of the present invention include, but are not limited to, leukaemia, breast cancer, skin cancer, bone cancer, prostate cancer, liver cancer, lung cancer, brain cancer, cancer of the larynx, gallbladder, pancreas, rectum, parathyroid, thyroid, adrenal, neural tissue, head and neck, colon, stomach, bronchi, kidneys, basal cell carcinoma, squamous cell carcinoma of both ulcerating and papillary type, metastatic skin carcinoma, osteo sarcoma, Ewing's sarcoma, veticulum cell sarcoma, myeloma, giant cell tumour, small-cell lung tumour, gallstones, islet cell tumour, primary brain tumour, acute and chronic lymphocytic and granulocytic tumours, hairy-cell tumour, adenoma, hyperplasia, medullary carcinoma, pheochromocytoma, mucosal neuromas, intestinal ganglioneuromas, hyperplastic corneal nerve tumour, marfanoid habitus tumour, Wilms' tumour, seminoma, ovarian tumour, leiomyomata, cervical dysplasia and in situ carcinoma, neuroblastoma, retinoblastoma, soft tissue sarcoma, malignant carcinoid, topical skin lesion, mycosis fungoide, rhabdomyosarcoma, Kaposi's sarcoma, osteogenic and other sarcoma, malignant hypercalcemia, renal cell tumour, polycythermia vera, adenocarcinoma, glioblastoma multiforme, leukemias, lymphomas, malignant melanomas, epidermoid carcinomas, and other carcinomas and sarcomas.

Generally, cells in benign tumours retain their differentiated features and do not divide in a completely uncontrolled manner. A benign tumour is usually localized and nonmetastatic. Specific types of benign tumours that can be prevented by the pharmaceutical composition of the present invention include hemangiomas, hepatocellular adenoma, cavernous haemangioma, focal nodular hyperplasia, acoustic neuromas, neurofibroma, bile duct adenoma, bile duct cystanoma, fibroma, lipomas, leiomyomas, mesotheliomas, teratomas, myxomas, nodular regenerative hyperplasia, trachomas and pyogenic granulomas. In the case of malignant tumours, cells become undifferentiated, do not respond to the body's growth control signals, and multiply in an uncontrolled manner. Malignant tumours are invasive and capable of spreading to distant sites (metastasizing). Malignant tumours are generally divided into two categories: primary and secondary. Primary tumours arise directly from the tissue in which they are found. Secondary tumours, or metastases, are tumours that originated elsewhere in the body but have now spread to distant organs. Common routes for metastasis are direct growth into adjacent structures, spread through the vascular or lymphatic systems, and tracking along tissue planes and body spaces (peritoneal fluid, cerebrospinal fluid, etc.).

In a further preferred embodiment of the invention the cancer to be prevented by the pharmaceutical composition according to the invention is prostate cancer.

In one embodiment, the pharmaceutical composition of the invention can be used for prevention of diseases associated with undesired and uncontrolled cell proliferation by administering to a subject suffering from uncontrolled cell proliferation a therapeutically effective amount of the composition comprising phenylbutyric acid.

One preferred embodiment relates to a pharmaceutical composition according to the invention, comprising said phenylbutyric acid, prodrug, derivative, codrug, co-crystal, solvate or pharmaceutically acceptable salt thereof for use in the prevention of cancer by administration of between 80 to 4000 mg/day, or 200 to 2000 mg/day or of a maximum of 1000 mg/day of the above mentioned phenylbutyric acid.

Preferably, in the pharmaceutical composition used according to the invention the treatment is applied by oral administration of the pharmaceutical composition, preferably is applied by once or twice daily oral administration of the pharmaceutical composition.

It is also preferable if the treatment is applied to a child, preferably is applied to a child (weighing less than 20 kg) by administration of an amount of said phenylbutyric acid as active component less than 450 mg/kg/day.

Alternatively, in the pharmaceutical composition used according to the invention the treatment is applied by administration of said phenylbutyric acid as active component by a maximum of between 750 and 250 mg/day, preferably of between 600 and 400 mg/day, most preferably of approximately or a maximum of 500 mg/day.

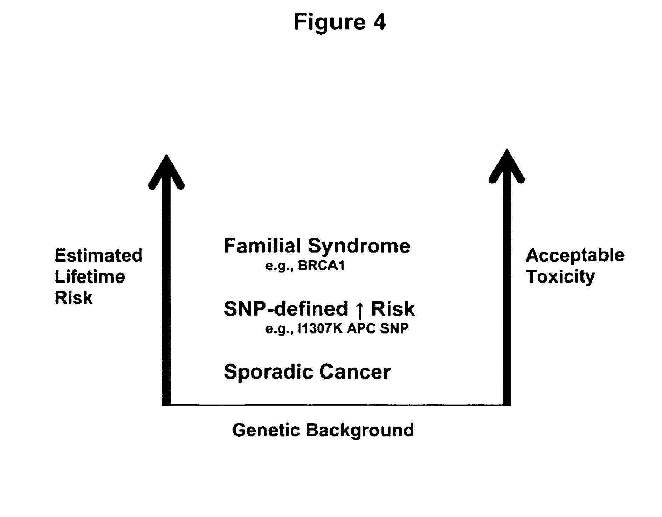

The particular dosage of the inhibitor to be used will depend on the individual risk factors, the route of administration, and related factors that can be determined by the attending physician. Generally, acceptable and effective daily doses are amounts sufficient to effectively reduce the risk of carcinogenesis. Normally the toxicity of chemopreventive agents represents a major hurdle for a adequate dosing regimen. As depicted in Figure 4 the qualitative ranking of cancer risk reduction interventions is based on the toxicity of the respective chemopreventive agent. The level of toxicity that would be acceptable to the patient increases with conditions that lead to a higher risk of cancer. Due to the high safety of phenylbutyrate, proven by the long-time clinical use, the toxicity does not represent a dosage-limiting problem for said chemopreventive agent. Accordingly the phenylbutyric acid according to the invention can be given also in higher dosages which are required for subjects with an increased risk for carcinogenesis.

In one embodiment of the invention the pharmaceutical composition of the invention is used in an intermittent therapeutical approach such as "SITEP" (Short-term Intermittent Therapy to Eliminate Premalignancy) (see figure 4). In this therapeutic approach the chemopreventive pharmaceutical composition is delivered systemically, but only cause apoptosis in the premalignant cells without harming the normal cells, substantially decreasing toxicity. Furthermore, treatments are given for short periods of time, followed by intervals of "drug-off" periods. Thus, the treatment regimen is intermittent. This intermittent approach potentially decreases the long-term toxicities associated with conventional chemoprevention approaches.

In a preferred embodiment of the invention the aforementioned SITEP approach is used for pharmaceutical compositions comprising a chemoprotective agent or a mixture thereof with increased toxicity.

In another preferred embodiment the prevention of cancer by the pharmaceutical composition comprising phenylbutyric acid is done by treating the patient by administration of an amount of phenylbutyric acid of less than 9.9 g/m²/day.

In another preferred embodiment the pharmaceutical composition is given by oral administration, preferably by once or twice daily oral administration.

A highly important aspect of the invention rests in that the use of the pharmaceutical composition comprising phenylbutyric acid, is done in a way to ensure a constant level (concentration) of the active component phenyl-butyric acid, prodrug, codrug, co-crystal, derivative or physiologically acceptable salt thereof in the blood.

Accordingly, in another preferred embodiment the phenylbutyric acid used in the prevention of cancer is comprised in a pharmaceutical composition being a prolonged release oral pharmaceutical unit dosage form, preferably comprising a controlled release agent, more preferably selected from the group consisting of cellulose derivatives, polyvinyl chloride, polyvinyl acetate, polyethylene, or polymers or co-polymers of acrylic acid esters and methyl-acrylic acid esters, even more preferably selected from cellulose derivatives like ethylcellulose, methylcellulose, ethylhydroxyethylcellulose, ethylcellulose, hyhydroxypropylmethylcellulose (HPMC) or hydroxypropylcellulose (HPC).

Other options could include pharmaceutical composition being oral pharmaceutical unit dosage forms with an enteric coating or being based on nanoparticles to ensure a constant blood level of the phenylbutyric acid. Very preferred embodiments of the oral pharmaceutical composition to be used according to the invention and comprising 4-phenylbutyric acid, a derivative, prodrug, codrug, cocrystal or a physiologically acceptable salt thereof are disclosed in EP 1 427 396 A1 (W02003/022253), the content of which forming herewith part of the description/disclosure of the current invention. In using this oral pharmaceutical composition, the active component phenylbutyric acid is applied twice daily with 40 to 2000 mg/day, or 100 to 1000 mg/day or twice daily with 250 mg/day of the active component (like 4-phenyl-butyric acid or sodium phenylbutyrate).

In another aspect the invention provides a pharmaceutical composition which further comprises an active component B which is selected from the group consisting of a chemoprotective agent, an anti-inflammatory agent, a vitamin, a dietary mineral, and an antioxidant.

The simultaneous administration of multiple chemopreventive agents can increase the efficacy and reduce toxicity. Such an approach preferably uses differences in the mechanisms of cancer prevention among the agents to increase the preventive activity. Further the increased efficacy achieves desirable levels of cancer inhibition at lower and presumably less toxic doses of the individual agents.

In a further embodiment the pharmaceutical composition according to the invention comprises a chemoprotective agent as component B which is selected from the group consisting of short chain fatty acids, NSAIDs, HDAC inhibitors not related to phenylbutyric acid, polyphenols, stilbenes, flavones and flavinoids, vitamin D derivatives, curcumin derivatives, organosulfur compounds, organoselenium compounds, polyunsaturated fatty acids and esters thereof, carotenoids, chalcone derivatives, steroids, cyclic triterpenes, phenylpropene derivatives, monoterpenes and chemopreventive phytochemicals.

Non-limited examples of short chain fatty acids are propionic acid, n-butyric acid, valproic acid, AN-9 (pivaloyl-oxymethyl butyrate), 2-n-propyl-4-pentynoic acid, valproyl hydroxamic acid, N-(1-hydroxyethyl)-valpromide, 2-hydroxypropyl-valpromide and N-methoxy valpromide and alpha-lipoic acid. The structures of selected short chain fatty acids are given in the following table.

| **Name** | **Formula** | **Comment/Reference** |
|---|---|---|
| **Short chain fatty acids** | | |
| Valproic acid | 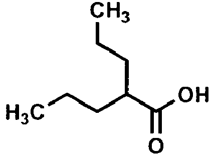 | |
| AN-9 (pivaloyloxymethyl butyrate) | 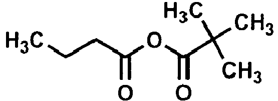 | Prodrug of butyric acid |
| Butyric acid | 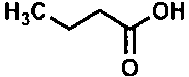 | |
| 2-n-propyl-4-pentynoic acid | 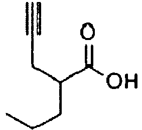 | Analogue of valproic acid |
| Valproyl hydroxamic acid | 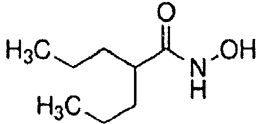 | |
| N-(1-hydroxyethyl)-valpromide | 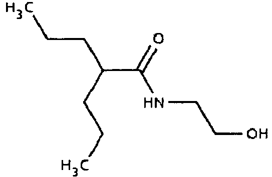 | |
| 2-hydroxypropyl-valpromide | 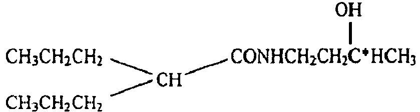 | |
| N-methoxy valpromide | 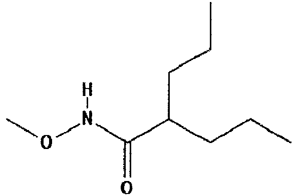 | |

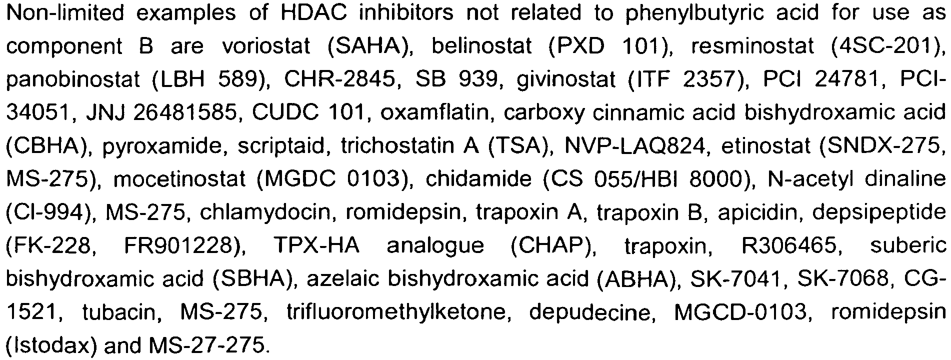

Non-limited examples of NSAIDs for use as component B are acetylsalicylic acid, para-aminosalicylic acid ethenzamide, salicylamide, diflunisal, salsol, salsalate, salicin, methylsalicylate, trisalicylates, meclofenamic acid, niflumic acid, flufenamic acid, mefenamic acid, etofenamate, tolfenamic acid, bufexamac, diclofenac, lonazolac, acemetacin, indomethacin, tolmetin, nalbumetone, sulindac, sulindac sulphide, ibuprofen, flurbiprofen, ketoprofen, fenoprofen, fenbufen, tiaprofenic acid, ketorolac, etodolac, bromfenac, metamizil, propyphenazone, paracetamol, phenidine, propacetamol, kebuzone, mofebutazone, oxyphenbutazone, phenylbutazone, naproxen, tolmetin, oxaprosin, piroxicam, tenoxicam, isoxicam, lornoxicam, nimesulide, NS-398, flosulid, L-745337, rofecoxib, celecoxib, etoricoxib, parecoxib, lumiracoxib, valdecoxib. For further examples see Christoph and Buschmann, 2002 (Cyclogenase inhibition: From NSAIDs to selective COX-2 inhibitors. In: Sundermann B, Buschmann H; Christoph T; Friderichs E; Maul C (Eds.): Analgesics: From Chemistry and Pharmacology to Clinical Application, page 13-126, Weinheim: Wiley-VCH 2002) which is incorporated herein by reference.

Non-limited examples of polyphenols are (-)-epicatechin (EC), (-)-epicatechin-3-gallate (ECG), (-)-epigallocatechin-3-gallate (EGCG), (-)-epigallocatechin (EGC), (+)-catechin" (*)-gallocatechin, (-)-robinetinidol, (+)-fisetinidol, (-)-fisetinidol, (+)-afzelechin, (+)-epiafzelechin and (-)-epiafzelechin; SU11274, theacetrin, theaflavin, theasinesin, theanaphthoquinone, olongtheanin, elagic acid, punigalacin, pinoresinol, lariciresinol, secoisolariciresinol, matairesinol, hydroxymatairesinol, syringaresinol and sesamin.

Non-limited examples of stilbenes are resveratrol, resveratrol-triacetate, digalloylresveratrol, pterostilbene, hydroxystilbene, dihydroxystilbene, trihydroxystilbene, tetrahydroxystilbene, pentahydroxystilbene, trimethoxystilbene, tetramethoxystilbene, pentamethoxystilbene, KITC, N-hydroxy-N'-(trimethoxphenyl)-trimethoxy-benzamidine, gloriosaol A, gloriasol B, gloriasol C, luteolin and piceatannol. For further examples see Fulda 2010 (Drug Discovery Today 15: 1590-1611) which is incorporated herein by reference.

Non-limited examples of flavones and flavinoids are quercetin, apigenin, biapigenin, phenoxodiol (NV-128), triphendiol, NV-143 (ME-143), naringenin, naringin, narirutin, hesperetin, hesperidin, daidzein, dihydro-daidzein, glycitein, genistein, dihydro-genistein, O-DMA, myricetin, campherol, baicalin, pelargonidin, cyanidin, dephinidin, 4-bromoflavone.

Non-limited examples of vitamin D and vitamin D derivatives are vitamin D2, 1α,25(OH)₂D₃, 22-oxa-1α,25(OH)₂D₃ (OCT), 19-nor-1α,25(OH)₂D₂, 1α(OH)₂D₃, calcipotriol, 1α,25(OH)₂-16-ene-23-yne-D₃, KH1060, EB1089, MC1288, ED-71, 2MbisP and Gemini analogs of vitamin D3 such as BXL 01-0108, BXL 01-0101, BXL 01-0098, BXL 01-0120, BXL 01-0117, BXL 01-0114, Gemini 2198, Gemini 3582, Gemini 0084, Gemini 0088, Gemini 0072 and Gemini 0097. For further examples see Brown et al. 2008 (Mol Aspects Medicine 29: 433-452) which is incorporated herein by reference.

Non-limited examples of curcumin derivatives are curcumin, tetrahydrocurcumin, dihydrocurcumin, hexahydrocurcumin, hexahydrocurcuminol, curcuminglucucronide, demethoxycurcumin, bisdemethoxycurcumin, cyclocurcumin, curcumin sulphate, cassumuin A, cassumuin B, [6]-paradol, oregonin, yakuchinone A, chlorogenic acid, ferulic acid, [6]-gingerol, dehydrozingerone, For further examples see Anand et al. 2008 (Biochem. Pharmacol. 76: 1590-1611) which is incorporated herein by reference.

Non-limited examples of organosulfur compounds are diallyldisulfide, diallylsulfide, 1-isothiocyanato-4-methylsulfinylbutane (sulphoraphane), allicin, benzyl isothiocyanate, alliin, oltipraz, anethole dithiolethione (ADT), 6-HITC, anethole trithione, 1,2-dithiole-3-thione, γ-glutamyl-S-allylcysteine, γ-glutamyl-S-trans-1-propenylcysteine, ajoene, S-allyl cysteine, S-allylmercapto cysteine, phenyl isothiocyanate and diallyl trisulfide.

Non-limited examples of organoselenium compounds are benzyl seleno cyanide, benzyl selenocyanate (BSC), and 1,4-phenylene bis(methylene) selenocyanate.

Non-limited examples of polyunsaturated fatty acids and esters thereof are ω-3 or ω-6 polyunsuturated fatty acids (PUFAs) such as α-linolenic acid, docosahexaenoic acid (DHA), DHA ethyl ester, hexadecatrienoic acid (HTA), stearidonic acid, eicosatetraenoic acid (ETA), eicosapentaenoic acid (EPA), heneicosapentaenoic acid (HPA), docosapentaenoic acid (DPA), tetracosapentaenoic acid, tetracosahexaenoic acid and eicosapentaenoic acid.

Non-limited examples of carotenoids are zeaxanthin, β-carotene, lutein, β-cryptoxanthine, lycopene, lycopersene, phytofluene, hexahydrolycopene, torulene, α-zeacarotene, alloxanthin, cynthiaxanthin, pectenoxanthin, cryptomonaxanthin, crustaxanthin, gazaniaxanthin, OH-chlorobactene, loroxanthin β, lycoxanthin γ, rhodopin, rhodopinol, and saproxanthin.

Non-limited examples of chalcone derivatives are chalcone, naringenin chalcone, phloridzin, myrigalone B, 2-hydroxychalcone, 4-hydroxychalcone, 2,4-dihydroxychalcone, 2-hydroxy-4-methoxychalcone, xanthohumol B, xanthohumol D, dihydroxyxanthohumol, isobavachalcon, robteine, buteine, licochalcone A, isoliquiritigenin, 1,2-dihydroparatocarpin A, isoliquiritin apioside, xanthohumol, broussochalcone A, phloretin, isobutrin, desmethylxanthohumol, asebogenin, isoliquiritigenin 2'-methyl ether, ON-III, licochalcone B, licochalcone D, cardamonin, flavokawain A, flavokawain B, hydroxysafflor yellow A, hydroxyderricin, uvaretin, isouvaretin, diuvaretin, isolespeol, licochalcone E, xanthoangelol and pedicine.

Non-limited examples of or steroids are diosgenin, oleandrin, oleanolic acid and guggulsterone,

Non-limited examples of cyclic triterpenes are glycyrrhizin, glycyrrhetinic acid, 18-β-glycyrrhetinic acid, bryonolic acid, betulinic acid, lupeol, celastrol, ursolic acid, acetyl-11-keto-β-boswellic acid (AKBA), α-boswellic acid, β-boswellic acid, 11-keto-β-boswellic acid, ganoderic acid C, ganoderic acid D, ginsenoside Rh2, ginsenoside Rf, ginsenoside Rg3, dihidrocucurbitacin B, cucurbitacin R, cucurbitacin B, cucurbitacin A, cucurbitacin E, cucurbitacin D, cucurbitacin Q (picracin), cucurbitacin R diglycoside, demethylzeylasteral, wilforic acid A, 24-metyllanosta-8,24(31)diene-3β,22ζ-diol, (22S,24R)24-metyllanosta-8-en-28-epoxy-3β,28ζ-diol, (22R,24S)24-metyllanosta-8-en-28-epoxy-3β,28ζ-diol, 17α-23-(E)-Dammara-20,23-diene-3β,25-diol, 2,3-dihydroxy-1,3,5(10),7-tetraene-6α(1'-hydroxyethyl)-24-nor-D,A-friedooleane-29-oic acid, pristimerin, tripterygone, oleanolic acod. 3-epikatonic acid, triptotriterpenonic acid A, 2α,3β-dihydroxy-olean-12-ene-22,29-lactone, kaikasaponin I, podocarpaside, soyasaponin III, astrasiversianin II, astrasiversianin I, astragaloside I, astragaloside II, astragaloside IV, astragaloside VI, cyclocanthoside E, cyclocanthoside G, avicin G, buddlejasaponin IV, momilactone B, carnosol, carnosic acid, impressic acid and lantanolic acid. For further examples see Rios et al. 2010 (J. Ethnopharmacol. 128: 1-14) which is incorporated herein by reference.

Non-limited examples of phenylpropene derivatives are 1'-acetoxychavicol acetate, eugenol, chavicol, safrole and estragole.

Non-limited examples of monoterpenes are limonene, pinenene, menthol, geraniol, camphene, sabinene, cadinine, perillyl alcohol (PA), geranial, neral, nerol, carvone, 1,8-cineol, carvacrol, thymol, and alpha-terpineol.

Non-limited examples for chemopreventive phytochemicals are yakuchinone A, yakuchinone B, capsaicin, dibenzoylmethane, piperine, kahweol, grayanotoxan, kauran, atisan, giberellan, indiruibin-3'-monoxime, caffeic acid, caffeic acid phenethyl ester (CAPE), emodin, linalol, quinic acid, garcinol, sesamin, theaflavin-3,3'-digallate, sanguinarine, silymarin, mangostin, mangiferin, butein, berberine, glabridin, carnosol, β-lapachone, evodiamine, wogonin, tanshinones IIA, tanshinones I, carnosol, zerumbone, sulforaphane, phytic acid, ascorbic acid, anethole, indole 3-carbinol, thymoquinone and plumbagin.

Non-limited examples of vitamine E derivatives are α-tocotrienol, β-tocotrienol, γ-tocotrienol, δ-tocotrienol, chalcone, α-tocopherol, β-tocopherol, γ-tocopherol, ζ-tocopherol, α-tocomoneol, β-tocomoneol, γ-tocomoneol, δ-tocomoneol, α-MDT, β-met, γ-MDT, δ-MDT, whereby the tocotrienol compounds are preferred.

In a preferred embodiment the pharmaceutical composition according to the invention comprises one of the following combinations:
● a salt of 4-phenylbutyrate with a positively charged ion of component B; including a respective polymorph, hydrate or solvate of the salt;
● a salt of 4-phenylbutyrate with a positively charged ion of component B;
● a polymorph of a salt of 4-phenylbutyrate with a positively charged ion of component B;
● a hydrate of a salt of 4-phenylbutyrate with a positively charged ion of an component B;
● a solvate of a salt of 4-phenylbutyrate with a positively charged ion of component B.

"Polymorphs" in general are described in "Polymorphism in Pharmaceutical Solids", Harry G. Brittain (ed.) Drugs and the Pharmaceutical Sciences 192 (1999).

In a most preferred embodiment the pharmaceutical composition according to the invention comprises one of the following combinations:
● a salt of 4-phenylbutyrate with a positively charged ion of a chemopreventive agent; including a respective polymorph, hydrate or solvate of the salt;
● a salt of 4-phenylbutyrate with a positively charged ion of a chemopreventive agent;
● a polymorph of a salt of 4-phenylbutyrate with a positively charged ion of a chemopreventive agent;
● a hydrate of a salt of 4-phenylbutyrate with a positively charged ion of a chemopreventive agent;
● a solvate of a salt of 4-phenylbutyrate with a positively charged ion of a chemopreventive agent;

In a further preferred embodiment the pharmaceutical composition according to the invention comprises one of the following combinations:
● a co-crystal of 4-phenylbutyric acid and component B as co-crystal former including a respective polymorph, hydrate or solvate of the co-crystal;
● a co-crystal of 4-phenylbutyric acid and component B as co-crystal former;
● a polymorph of a co-crystal of 4-phenylbutyric acid and component B as co-crystal former;
● a hydrate of a co-crystal of 4-phenylbutyric acid and component B as co-crystal former; or
● a solvate of a co-crystal of 4-phenylbutyric acid and component B as co-crystal former.

In the scientific literature there is currently some discussion on the proper use of the word co-crystal (see for example Desiraju, CrystEngComm, 2003, 5(82), 466-467 and Dunitz, CrystEngComm, 2003, 5(91), 506). A recent article by Zaworotko (Zaworotko, Crystal Growth & Design, Vol. 7, No. 1, 2007, 4-9) gives a definition of co-crystal which is in line with the definition given above.

A "component B as cocrystal former" as used herein is defined as any compound B, which is selected from the group consisting of short chain fatty acids, NSAIDs, HDAC inhibitors not related to phenylbutyric acid, polyphenols, stilbenes, flavones and flavinoids, vitamin D derivatives, curcumin derivatives, organosulfur compounds, organoselenium compounds, polyunsaturated fatty acids and esters thereof, carotenoids, chalcone derivatives, steroids, cyclic triterpenes, phenylpropene derivatives, monoterpenes and chemopreventive phytochemicals, being preferably a polyphenol, which is able to form a co-crystal with 4-phenylbutyric acid or its salts.

In a preferred embodiment the pharmaceutical composition comprises one of the above listed co-crystals, wherein the difference in pKA between 4-phenylbutyric acid and component B does not exceed 2.7.

In a further embodiment the pharmaceutical composition according to the invention comprises one of the following combinations:
● a co-crystal of a salt of 4-phenylbutyrate with a positively charged ion of component B and at least one organic molecule as co-crystal former including a respective polymorph, hydrate or solvate of the co-crystal;
● a co-crystal of a salt of 4-phenylbutyrate with a positively charged ion of component B and at least one organic molecule as co-crystal former;
● a polymorph of a co-crystal of a salt of 4-phenylbutyrate with a positively charged ion of component B and at least one organic molecule as co-crystal former;
● a hydrate a co-crystal of a salt of 4-phenylbutyrate with a positively charged ion of component B and at least one organic molecule as co-crystal former;
   or
● a solvate a co-crystal of a salt of 4-phenylbutyrate with a positively charged ion of component B and at least one organic molecule as co-crystal former.

In a further aspect the invention provides a pharmaceutical composition comprising a codrug, a pharmaceutically acceptable salt, solvate or prodrug thereof, which comprises 4-phenylbutyric acid and chemoprotective component B selected from the group consisting of HDAC inhibitors different from phenylbutyric acid, short chain fatty acids, NSAIDs, polyphenols, stilbenes, flavones and flavinoids, vitamin D derivatives, curcumin derivatives, organosulfur compounds, organoselenium compounds, polyunsaturated fatty acids and esters thereof, carotenoids, chalcone derivatives, steroids, cyclic triterpenes, phenylpropene derivatives, monoterpenes and chemopreventive phytochemicals.

In a preferred embodiment said pharmaceutical composition comprising a codrug, a pharmaceutically acceptable salt, or prodrug thereof, wherein the codrug comprises:
a) at least two components, including 4-phenylbutyric acid as a first component and a second component B selected from the group consisting of HDAC inhibitors different from phenylbutyric acid, short chain fatty acids, NSAIDs, polyphenols, stilbenes, flavones and flavinoids, vitamin D derivatives, curcumin derivatives, organosulfur compounds, organoselenium compounds, polyunsaturated fatty acids and esters thereof, carotenoids, chalcone derivatives, steroids, cyclic triterpenes, phenylpropene derivatives, monoterpenes and chemopreventive phytochemicals;
b) a linkage covalently linking said at least two components to form said codrug, said linkage is cleaved under physiological conditions after the codrug has been administered to the subject to regenerate said components.

Yet another aspect of the invention provides a pharmaceutical composition comprising a codrug, wherein the codrug has the following structural formula:

R₁ - L- (R₂)ₙ

wherein the first component is R₁;
the linker L
the second component is R₂;
R₁ represents, independently, a residue of a 4-phenylbutyric acid including 4-phenylbutyrate, a 4-phenylbutamido group, or a 4-phenylbutoxy group;
R₂ represents a residue of a compound B selected from short chain fatty acids, HDAC inhibitors different from phenylbutyric acid, NSAIDs, polyphenols, stilbenes, flavones and flavinoids, vitamin D derivatives, curcumin derivatives, organosulfur compounds, organoselenium compounds, polyunsaturated fatty acids and esters thereof, carotenoids, chalcone derivatives, steroids, cyclic triterpenes, phenylpropene derivatives, monoterpenes and chemopreventive phytochemicals;
n is an integer of from 1 to 4;
and L is selected from a direct bond and a linking group.

The linker L may be either a direct bond between individual components, or it may include a linking group. The first and second components of the codrugs of the present invention may be linked via reversible covalent bonds such as ester, amide, carbamate, carbonate, cyclic ketal, thioester, thioamide, thiocarbamate, thiocarbonate, xanthate and phosphate ester bonds, so that, at the required site in the body, they are cleaved to regenerate the active forms of the constituent pharmaceutically active agents.

The covalent bonds between residues include a bonding structure such as: 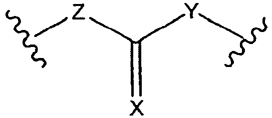 wherein Z is O, N, -CH₂-, -CH₂-O- or -CH₂-S-, Y is O, or N, and X is or S.

In a further embodiment, the linker comprises an azo group, which in a preferred embodiment is a linking group according the following formula: 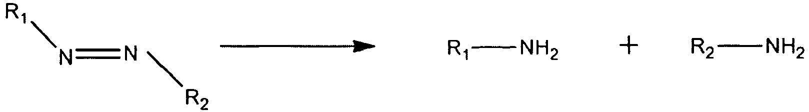 wherein R₁ and R₂ represent the above defined residues.

The azo group can be cleaved by the enzyme azoreductase to yield the respective amines.

In another embodiment, the linker comprises an carbonate ester moiety, which in a preferred embodiment is a linking group according the following formula: 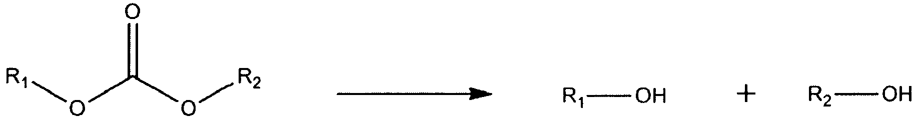 wherein R₁ and R₂ represent the above defined residues.

As shown above, the cleavage of the carbonate ester moiety yields two alcohol compounds.

In a further embodiment, the linker comprises a carbamate moiety, which in a preferred embodiment is a linking group according the following formula: 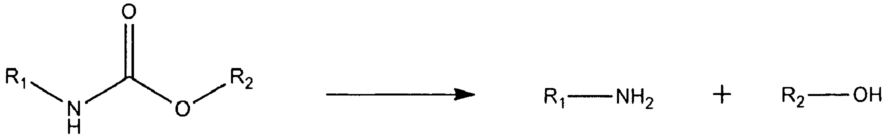 wherein R₁ and R₂ represent the above defined residues.

As shown above, the cleavage of the carbamate moiety yields an amine and an alcohol.

In another embodiment, the linker comprises a dicarboxylic acid ester moiety, which in a preferred embodiment is a linking group according the following formula: 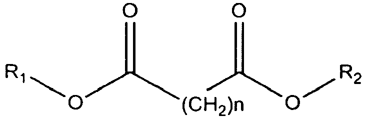 wherein R₁ and R₂ represent the above defined residues and n is from 1 to 12.

In a further embodiment, the linker comprises a linear dihydroxy residue, which in a preferred embodiment is a linking group according the following formula: 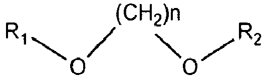 wherein R₁ and R₂ represent the above defined residues and n is from 1 to 12.

In one embodiment, the linker comprises a linear diamino moiety, which in a preferred embodiment is a linking group according the following formula: 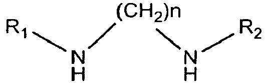 wherein R₁ and R₂ represent the above defined residues and n is from 1 to 12.

The rate of cleavage of the individual component residues can be controlled by the type of bond, the choice of constituent moieties, and the physical form of the codrug. The lability of the selected bond type may be enzyme-specific. In some embodiments according to the present invention, the bond is selectively labile in the presence of an esterase. In other embodiments of the invention, the bond is chemically labile, e.g., to acid- or base-catalyzed hydrolysis.

The physiologically labile linkage may be any linkage that is labile under conditions approximating those found in physiologic fluids, such as is found in the gastrointestinal tract, the blood, the liquor, the dermis or viable epidermis. The linkage may be a direct bond (for instance, ester, amide, carbamate, carbonate, cyclic ketal, thioester, thioamide, thiocarbamate, thiocarbonate, xanthate, phosphate ester, sulfonate, or a sulfamate linkage) or may be a linking group (for instance a C₁-C₁₂ dialcohol, a C₁-C₁₂ hydroxylalkanoic acid, a C₁-C₁₂ hydroxyalkylamine, a C₁-C₁₂ diacid, a C₁-C₁₂ amino acid, or a C₁-C₁₂ diamine). Especially preferred linkages are direct amide, ester, carbonate, carbamate, and sulfamate linkages, and linkages via succinic acid, salicylic acid, diglycolic acid, oxa acids, oxamethylene, and halides thereof. The linkages are labile under physiologic conditions, which generally mean pH of about 6 to about 8. The lability of the linkages depends upon the particular type of linkage, the precise pH and ionic strength of the physiologic fluid, and the presence or absence of enzymes that tend to catalyze hydrolysis reactions in vivo. In general, lability of the linkage in vivo is measured relative to the stability of the linkage when the codrug has not been solubilized in a physiologic fluid.

Thus, while some codrugs according to the present invention may be relatively stable in some physiologic fluids, nonetheless, they are relatively vulnerable to hydrolysis in vivo (or in vitro, when dissolved in physiologic fluids, whether naturally occurring or simulated) as compared to when they are neat or dissolved in non-physiologic fluids (e.g., nonaqueous solvents such as acetone).

Thus, the labile linkages are such that, when the codrug is dissolved in an aqueous solution, especially a physiologic fluid such as blood or liquor, the reaction is driven to the hydrolysis products, which include the components set forth above.

Codrugs for preparation of topical or transdermal compositions according to the present invention may be synthesized in the manner as known for the skilled person. In general, where the first and second components are to be directly linked, the 4-phenylbutyric acid as first component is condensed with the second component B under conditions suitable for forming a linkage that is labile under physiologic conditions. In some cases it is necessary to block some reactive groups on one, the other, or both of the components. Where the two components are to be covalently linked via a linker, such as oxamethylene, succinic acid, or diglycolic acid, it is advantageous to first condense the 4-phenylbutyric acid with the linker. In some cases it is advantageous to perform the reaction in a suitable solvent, such as acetonitrile, in the presence of suitable catalysts, such as carbodiimides including EDCI (1-ethyl-3-(3-dimethylaminopropyl)-carbodiimide) and DCC (DCC: dicyclohexylcarbodiimide), or under conditions suitable to drive off water of condensation or other reaction products (e.g., reflux), or a combination of two or more thereof. After the 4-phenylbutyric acid residue is condensed with the linker, the combined first component and linker may then be condensed with the second component B. Again, in some cases it is advantageous to perform the reaction in a suitable solvent, such as acetonitrile, in the presence of suitable catalysts, such as carbodiimides including EDCI and DCC, or under conditions suitable to drive off , water of condensation or other reaction products (e.g., reflux), or a combination of two or more thereof. Where one or more active groups have been blocked, it may be advantageous to remove the blocking groups under selective conditions, however it may also be advantageous, where the hydrolysis product of the blocking group and the blocked group is physiologically benign, to leave the active groups blocked.

The person having skill in the art will recognize that, while diacids, dialcohols, amino acids, etc. are described above as being suitable linkers, other linkers are contemplated as being within the present invention. For instance, while the hydrolysis product of a codrug according to the present invention may comprise a diacid, the actual reagent used to make the linkage may be, for example, an acylhalide such as succinyl chloride. The person having skill in the art will recognize that other possible acid, alcohol, amino, sulfato, and sulfamoyl derivatives may be used as reagents to make the corresponding linkage.

Where the first and second components are to be directly linked via a covalent bond, essentially the same process is conducted, except that in this case there is no need for a step of adding a linker. The 4-phenylbutyric acid residue and the second component B are merely combined under conditions suitable for forming the covalent bond. In some cases it may be desirable to block certain active groups on one, the other, or both of the pharmaceutically active moieties. In some cases it may be desirable to use a suitable solvent, such as acetonitrile, a catalyst suitable to form the direct bond, such as carbodiimides including EDCI and DCC, or conditions designed to drive off water of condensation (e.g., reflux) or other reaction by-products.

The person having skill in the art will recognize that, while in most cases the 4-phenylbutyric acid and the second component B may be directly linked in their original form, it is possible for the active groups to be derivatized to increase their reactivity. For instance, where the second moiety is an alcohol (i.e., has a free hydroxyl group), the 4-phenylbutyric acid may be derivatized to form the corresponding acid halide, such as an acid chloride or an acid bromide. The person having skill in the art will recognize that other possibilities exist for increasing yield, lowering production costs, improving purity, etc. of the codrug according to the present invention by using conventionally derivatized starting materials to make codrugs according to the present invention.

Exemplary reaction schemes according to the present invention are illustrated in Schemes 1-4, below. These Schemes can be generalized by substituting other components B having at least one functional group that can form a covalent bond to another therapeutic agent having a similar or different functional group, either directly or indirectly through a pharmaceutically acceptable linker. The person of skill in the art will appreciate that these schemes also may be generalized by using other appropriate linkers.

Scheme 1: R₁-COOH + R₂-OH → R₁-COO-R₂ = R₁-L-R₂

wherein L is an ester linker -COO-, R₁ is a phenylbutyric acid residue and R₂ is the residues of the component.

Scheme 2: R₁-COOH + R₂ -NH₂ → R₁-CONH-R₂ = R₁-L-R₂

wherein L is an amide linker -CONH- and R₁ and R₂ have the meaning as given above.

Scheme 3: Step 1: R₁-COOH + HO-L-CO-Prot → R₁-COO-L-CO-Prot

wherein Prot is a suitable reversible protecting group.
Step 2: R₁-COO-L-CO-Prot → R₁-COO-L-COOH
Step 3: R₁-COO-L-COOH + R₂-OH → R₁-COO-L-COOR₂
wherein L, R₁ and R₂ have the meaning as given above. 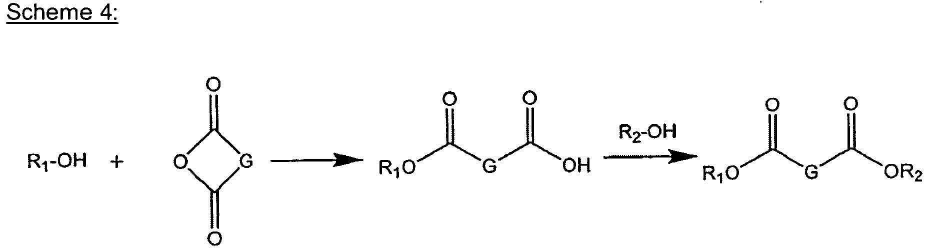 wherein R₁ and R₂ have the meanings set forth above and G is a direct bond, an C₁-C₄ alkylene, a C₂-C₄ alkenylene, a C₂-C₄ alkynylene, or a 1, 2-fused ring, and G together with the anhydride group completes a cyclic anhydride. Suitable anhydrides include succinic anhydride, glutaric anhydride, malefic anhydride, diglycolic anhydride, and phthalic anhydride.

In another aspect the invention relates to a method of preventing cancer by administering to a subject a pharmaceutical composition according to the invention.

### DEFINITIONS

Throughout the description and claims of this specification the word "comprise" and other forms of the word, such as "comprising" and "comprises," means including but not limited to, and is not intended to exclude, for example, other additives, components, integers, or steps.

As used herein, the term "pharmaceutical composition" refers to any composition comprising at least one pharmaceutically active component and at least one other component, as well as any product which results, directly or indirectly, from combination, complexation, or aggregation of any two or more of the components, from dissociation of one or more of the components, or from other types of reactions or interactions of one or more of the components. Accordingly, the term "pharmaceutical composition" as used herein may encompass, inter alia, any composition made by admixing a pharmaceutically active component and one or more pharmaceutically acceptable carriers.

As used in the context of the present invention the term "phenylbutyric acid" refers to all phenyl-substituted butyric acid isomers, such as 4-phenylbutyric acid, 3-phenylbutyric acid and 2-phenylbutyric acid, wherein the phenyl-residue can be unsubstituted or substituted with one ore more identical or different substituents.

Within the context of the present invention the term "derivative" is defined as any structural analogue or synthetic derivatives In the context of the present invention, the term "derivative" refers to a similar structure or structural analogue derived from the organic acid structure (unit) in question, i.e. a 4-phenylbutyrate derivative is understood to mean, for example, a compound which includes a 4-phenylbutoxy unit and therefore also encompasses a 4-phenylbutyramide analogue.

The term "prodrug" refers to compounds that are rapidly transformed in vivo to yield the phenylbutyric acid parent compound, for example by hydrolysis in blood. Functional groups that may be rapidly transformed, by metabolic cleavage, in vivo form a class of groups reactive with the carboxyl group of the compounds of this invention. They include, but are not limited to such groups as alkanoyl (such as acetyl, propanoyl, butanoyl, and the like), unsubstituted and substituted aroyl (such as benzoyl and substituted benzoyl), alkoxycarbonyl (such as ethoxycarbonyl), trialkylsilyl (such as trimethyl- and triethysilyl), monoesters formed with dicarboxylic acids (such as succinyl), and the like. Because of the ease with which the metabolically cleavable groups of the compounds of this invention are cleaved in vivo, the compounds bearing such groups act as pro-drugs. The compounds bearing the metabolically cleavable groups have the advantage that they may exhibit improved bioavailability as a result of enhanced solubility and/or rate of absorption conferred upon the parent compound by virtue of the presence of the metabolically cleavable group. A thorough discussion is provided in Prodrugs and Targeted Delivery: Towards Better ADME Properties (Methods and Principles in Medicinal Chemistry), Mannhold et al, Ed., Wiley-VCH (2010), which is incorporated herein by reference.

The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

In the context of the present invention, the term "pharmaceutically acceptable salt" comprises the salts prepared with non-toxic acids or bases, depending on the substituents present on the compounds. Since the phenylbutyric acid contain an acidic function, the corresponding salts can be obtained by addition of an organic or inorganic base to the compound in neutralized form possibly in the presence of a preferably inert solvent. Examples of addition salts of a base can be the sodium, potassium, calcium, ammonium, amino (organic), or magnesium salts.

The term "prevention of cancer" as used herein is synonymous with the terms "cancer prevention" and "chemoprevention" and relates to the use of a drug or a compound to interfere with the process of carcinogenesis thereby inhibiting or delaying carcinogenesis. The chemoprevention encompasses the so-called primary, secondary, or tertiary prevention. Primary chemoprevention focuses on preventing the development of precancerous lesions, secondary chemoprevention focuses on preventing the progression of these lesions to cancer and tertiary chemoprevention aims to prevent the recurrence or spread of a primary cancer. The term "tertiary chemoprevention" is synonymous to the term "chemoquiescence". The prevention as encompasses also the treatment of Carcinoma *in situ* (CIS). CIS is an early form of cancer that is defined by the absence of invasion of tumour cells into the surrounding tissue, usually before penetration through the basement membrane. In the TNM classification of malignant tumours, carcinoma in situ is reported as T*i*sN0M0 (Stage 0). Carcinoma in situ is synonymous with high-grade dysplasia in most organs.

In the context of the present invention a "drug" is synonymous to the term "medicament" and is defined as a chemical substance used in the treatment, cure, prevention, or diagnosis of disease or used to otherwise enhance physical or mental well-being. The chemical substance can be a pure chemical compound or a mixture of chemical compounds which can be an inorganic substance, an organic substance or a biomolecule.

"Co-crystal" as used herein is defined as a crystalline material comprising two or more compounds at ambient temperature (20 to 25°C, preferably 20°C), of which at least two are held together by weak interaction, wherein at least one of the compounds is a co-crystal former. Weak interaction is being defined as an interaction, which is neither ionic nor covalent and includes for example hydrogen bonds. Other modes of molecular recognition may also be present including, pi-stacking, guest-host complexation and van der Waals interactions. Of the interactions listed above, hydrogen-bonding is the dominant interaction in the formation of the co-crystal, (and a required interaction according to the present invention) whereby a non-covalent bond is formed between a hydrogen bond donor of one of the moieties and a hydrogen bond acceptor of the other. Solvates or salts of phenyl butyric acid and an organic compound that do not further comprise a co-crystal former are not co-crystals according to the present invention. The co-crystals may however, include one or more solvate molecules in the crystalline lattice. Just for the sake of clarity the distinction between crystalline salt and a co-crystal has to be stressed here. An API bound to another compound forming a salt by means of ionic interaction can be considered as one "compound" according to the invention, but it cannot be considered as two compounds by itself.

The term "codrug" as used herein means a compound, or a prodrug form thereof, comprising a first residue of phenylbutyric acid associated with a second residue, wherein both residues, in their unlinked forms (e.g., in the absence of the association), are biologically active. In preferred embodiments, one the second residue is a small molecule. The association between said residues is covalent and is either direct or indirect through a linker. The first residue can be the same or different from the second. The codrugs referred to herein may optionally be homocodrugs or heterocodrugs. A "homocodrug," also termed a "symmetrical codrug," refers to a codrug that produces, upon cleavage or dissociation, two or more molecules of phenylbutyric acid, and no other drug molecules, i.e., the homo codrug is composed primarily of two or more residues of a phenylbutyric acid, without incorporating a residue of a second drug. A "heterocodrug," also termed an "asymmetrical codrug," refers to a codrug that produces, upon cleavage or dissociation, residues of at least two different drugs.

### Legend to figures

- **Figure 1:**: Schematic diagram showing the pathogenesis for cancer of several organs indicating the window of opportunity (denoted as "gap") for chemopreventive treatment.
- **Figure 2:**: Selectivity of phenylbutyric acids with regard to inhibition of histone deacetylase inhibitors.

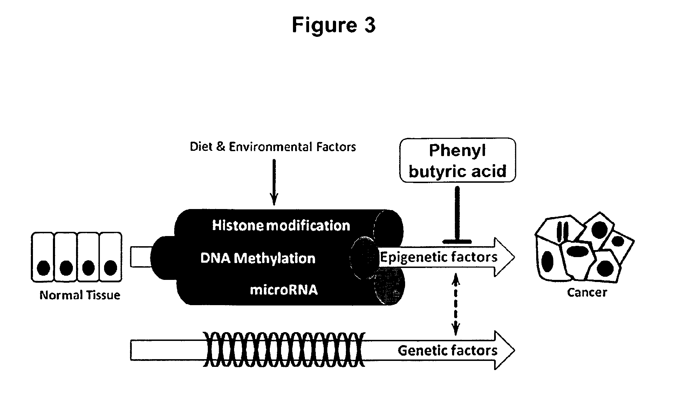

- **Figure 3:**: Chemopreventive effects of phenylbutyric acids on specific epigenetic alterations such as changes in DNA methylation, histone modifications and non-coding RNAs. These epigenetic alterations are influenced by diet and environmental factors.
- **Figure 4:**: Diagram outlining that an increased estimated lifetime risk for cancer beginning from sporadic cancer over SNP-defined risk to a familial syndrome allows a higher acceptable toxicity for a chemopreventive treatment.

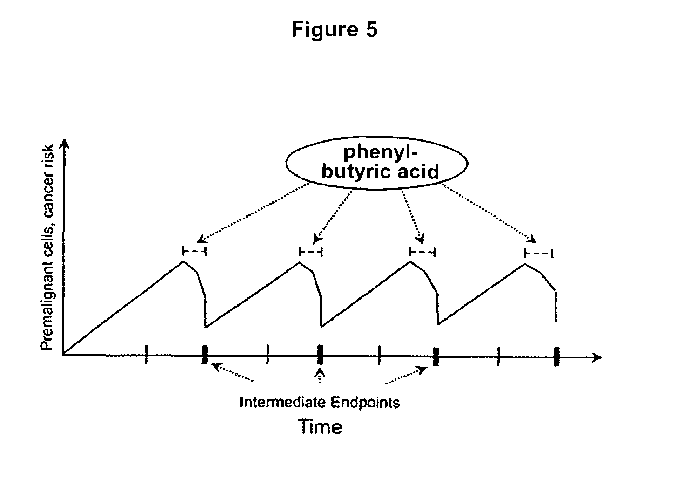

- **Figure 5:**: Schematic diagram showing the use of the composition comprising phenylbutyric acid by SITEP (Short-term Intermittent Therapy to Eliminate Premalignancy). As the number of premalignant cells increases, so does the patient's inherent cancer risk. By delivering short-term intermittent therapy, the number of premalignant cells is greatly decreased, thus decreasing the cancer risk. Performing therapy in this manner also enables the assessment of intermediate endpoints to assess efficacy, toxicity, and the potential development of resistance.

## Claims

1. A pharmaceutical composition comprising phenyl butyric acid, a derivative, a prodrug, solvate, codrug, co-crystal or a physiologically acceptable salt thereof for use in the prevention of cancer.

2. The pharmaceutical composition according to claim 1 wherein the phenyl-butyric acid is 4-phenylbutyric acid.

3. The pharmaceutical composition according to any of claims 1 or 2, wherein said prevention is a primary, secondary or tertiary prevention of cancer.

4. The pharmaceutical composition according to any of claims 1 to 3, wherein said pharmaceutical composition is administered to yield a maximum of 1000 mg/day of said phenylbutyric acid, derivative, solvent, codrug, co-crystal or pharmaceutically acceptable salt thereof.

5. The pharmaceutical composition used according to claim 4, wherein the pharmaceutical composition is administered orally, preferably once or twice daily.

6. The pharmaceutical composition used according to claims 4 or 5, wherein the treatment is applied to a child (weighing less than 20 kg) by administration of an amount of said phenylbutyric acid, derivative, prodrug, solvent, codrug, co-crystal or pharmaceutically acceptable salt thereof less than 450 mg/kg/day.

7. The pharmaceutical composition used according to claims 4 or 5, wherein the treatment is applied by administration of an amount of said phenylbutyric acid, derivative, prodrug, solvent, codrug, co-crystal or pharmaceutically acceptable salt of 250 and 750 mg/day, preferably of between 400 and 600 mg/day and most preferably of 500 mg/day.

8. The pharmaceutical composition according to any of claims 1 to 7 wherein said pharmaceutical composition is used in an intermittent therapeutical approach such as Short-term Intermittent Therapy to Eliminate Premalignancy (SITEP).

9. The pharmaceutical composition according to any of claims 1 to 8, wherein the composition comprises a physiologically acceptable salt of 4-phenylbutyric acid, more preferably is a physiologically acceptable salt of 4-phenylbutyric acid of the earth or earth alkaline type, most preferably is a lithium, sodium, magnesium or calcium, and most preferably the sodium salt of 4-phenylbutyric acid.

10. The pharmaceutical composition according to any of claims 1 to 9, wherein the pharmaceutical composition is a prolonged release oral pharmaceutical unit dosage form, preferably comprising a controlled release agent, preferably selected from the group consisting of cellulose derivatives, polyvinyl chloride, polyvinyl acetate, polyethylene, or polymers or co-polymers of acrylic acid esters and methyl-acrylic acid esters, more preferably selected from cellulose derivatives like ethylcellulose, methylcellulose, ethylhydroxyethylcellulose, ethylcellulose, hydroxypropylmethylcellulose (HPMC) or hydroxypropylcellulose (HPC).

11. The pharmaceutical composition according to any of claims 1 to 10, wherein the composition further comprises an component B which is a compound selected from the group consisting of a chemoprotective agent different from phenylbutyric acid, a vitamin, a dietary mineral, and an antioxidant.

12. The pharmaceutical composition according to claim 11 wherein the chemoprotective agent as component B is selected from the group consisting of HDAC inhibitors different from phenylbutyric acid, short chain fatty acids, NSAIDs, polyphenols, stilbenes, flavones and flavinoids, vitamin D derivatives, curcumin derivatives, organosulfur compounds, organoselenium compounds, polyunsaturated fatty acids and esters thereof, carotenoids, chalcone derivatives, steroids, cyclic triterpenes, phenylpropene derivatives, monoterpenes and chemopreventive phytochemicals.

13. A pharmaceutical composition according to any of the above claims, **characterized in that** the pharmaceutical composition comprises one of the following combinations:
a) a salt of 4-phenylbutyrate with a positively charged ion of component B including a respective polymorph, hydrate or solvate of the salt;
b) a salt of 4-phenylbutyrate with a positively charged ion of component B;
c) a polymorph of a salt of 4-phenylbutyrate with a positively charged ion of component B;
d) a hydrate of a salt of 4-phenylbutyrate with a positively charged ion of component B;
e) a solvate of a salt of 4-phenylbutyrate with a positively charged ion of component B.

14. A pharmaceutical composition according to any of claims claims 1 to 12, **characterized in that** the pharmaceutical comprises a co-crystal of one of the following combinations:
a) a co-crystal of 4-phenylbutyric acid and component B as co-crystal former including a respective polymorph, hydrate or solvate of the co-crystal;
b) a co-crystal of 4-phenylbutyric acid and component B as co-crystal former;
c) a polymorph of a co-crystal of 4-phenylbutyric acid and component B as co-crystal former;
d) a hydrate of a co-crystal of 4-phenylbutyric acid and component B as co-crystal former; or
e) a solvate of a co-crystal of 4-phenylbutyric acid and component B as co-crystal former.

15. The pharmaceutical composition according to any of claims 1 to 10 wherein the codrug comprises a residue of 4-phenylbutyric acid and a residue of component B selected from the group consisting of chemoprotective agent is selected from the group consisting of short chain fatty acids, HDAC inhibitors different from phenylbutyric acid, short chain fatty acids, NSAIDs, polyphenols, stilbenes, flavones and flavinoids, vitamin D derivatives, curcumin derivatives, organosulfur compounds, organoselenium compounds, polyunsaturated fatty acids and esters thereof, carotenoids, chalcone derivatives, steroids, cyclic triterpenes, phenylpropene derivatives, monoterpenes and chemopreventive phytochemicals.
